# EUROPEAN PATENT APPLICATION

(11) **EP 2 336 281 A1**
(43) Date of publication of application: **22.06.2011**
(21) Application number: 09015095.4
(22) Date of filing: 05.12.2009
(51) Int. Cl.: C11D 1/78, C11D 1/34, A61K 8/55, C07D 493/04, A61K 31/665

(54) **Use of anionic isosorbide derivatives (III)**

(71) Applicant: Cognis IP Management GmbH, 40589 Düsseldorf (DE)
(72) Inventor: Behler, Ansgar, 46240 Bottrop (DE); Breffa, Catherine, 40210 Düsseldorf (DE); Raths, Hans-Christian, 40789 Monheim (DE)

(57) **Abstract**

Isosorbide phosphates according to general formula (I)

Rₙ-PO(OX)ₘ (I)

wherein n und m have values from 1 to 3 with the proviso, that the sum from n + m is always 3, and X stands for a hydrogen atom, or an alkyl- or alkenyl moiety, and R is a compound according to the general formula (II) 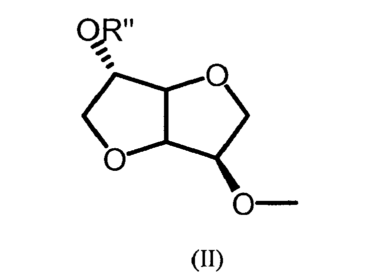 whereby in formula (II) R" represents a hydrogen atom or an alkyl- or alkenyl group with 1 to 22 C-atoms, which is linear or branched, saturated or unsaturated for the preparation of cleaners, detergents, personal care, cosmetic or pharmaceutical compositions, preferably in cleaners, personal care and cosmetic compositions.

## Description

The present application pertains to specific derivatives of isosorbide, and the use thereof in household products, like detergents or in cosmetic applications, preferably in personal cleaning applications, detergents and manual dish detergents.

The most widely used anionic surfactants in cleansing compositions are alkyl sulphates, polyoxyethylene alkyl sulphates and alkyl benzene sulphonates. These compounds are known to have a good foaming and deterging power. Due to their harshness, however, they are not desirable as components for cleansing compositions topically applied to human skin and hair. Their damaging effect particularly where young, tender or damaged skin is involved, has been the subject of intense study for many years.

On the other hand milder surfactants often suffer from the drawback that they do not provide high foam which is very important for the consumer. Therefore, there is a strong need for products which are not only very mild but also possess an excellent foaming power.

Isosorbide (or 1,4: 3,6-dianhydrosorbitol, see formula below) is the anhydride of sorbitol:

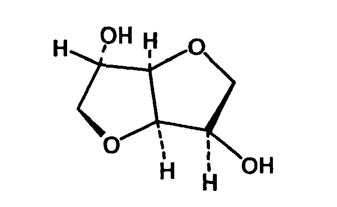

Upon heating sorbitol for example with concentrated sulfuric or hydrochloric acid, two molecules of water are eliminated with the formation of isosorbide. So far, these compounds are also known generally as dianhydrohexitols (including besides isosorbide also the isomers isomannide and isoidide).

Certain derivatives of isosorbide are known, especially esters or ether derivatives. Furthermore it is known to use isosorbide derivatives as additives in various applications, like detergents, cleansers or cosmetic compositions. US 2002/0174596 A1 discloses various isosorbide ethers as detergent for fuels. WO 01/0191949 A1 describes dimethylisosorbide as compound of a personal cleansing composition. In WO 99/45060 the use of certain isosorbide derivatives, including the phosphates is disclosed, but only as solvent for polymers and as plasticizer.

It was an objection of the present invention to find new uses for derivatives based on isosorbide chemistry. It was found that the phosphoric esters of isosorbide could be used with advantage in detergents, cleansers and related products, but preferably in personal cleaning applications.

The present application pertains in a first embodiment to the use of isosorbide phosphates according to general formula (I)

R-PO(OH)₂ (I)

whereby R is a compound according to the general formula (II) 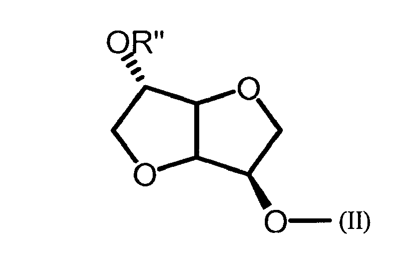 whereby in formula (II) R" represents a hydrogen atom or an alkyl- or alkenyl group with 1 to 22 C-atoms, which is linear or branched, saturated or unsaturated for the preparation of cleaners, detergents, personal care, cosmetic or pharmaceutical compositions, preferably in cleaners, personal care and cosmetic compositions Compounds according to formula (I) are isosorbide-phosphates or better isosorbide phosphate esters. The phosphates according to this invention are monoesters, with two hydroxyl-groups, attached to the phosphor atom each.

The compositions according to formula (I) can be prepared by known methods, for example by reaction of isosorbide with phosphor oxides in non-aqueous media. According to the method of preparation besides the wanted phosphates also by products could be present, in particular isomers of isosorbide and phosphates thereof.

The compounds following to formula (I) are useful for the preparation of all kind of detergents, cleansers and the like (solid, liquid or gel-like ones) or the use of these compounds in cosmetic or pharmaceutical compositions. Preferred is the use of compounds according to formula (I) in cleaners and here in particular for cleaners for hard surfaces, like kitchen or bathroom cleaner or dish washing detergents (manual and automatic), as well as cosmetic compositions or personal care applications too.

The isosorbide derivatives according to formula (I) then may be present in amounts from 0.1 up to 25 % by weight, dependent on the particular formulation. Preferably those detergents or cleansers will contain the isosorbide derivatives in amounts of 1 to 15 wt%, and most preferred from 5 to 10 wt%, based on the total weight of the cleanser or detergent.

The isosorbide derivatives according to formula (I) are particularly useful in home care applications, like detergents, and all kind of cleaners (kitchen, bathroom, hard surface, automotive or car cleansers, and multipurpose cleansers), as well as in dishwashing compositions (hand and automatic dish washing) and in personal care compositions, especially in hair and body cleansing formulations, but can also be used with advantage in cosmetic compositions, for example in shampoos, creams and the like.

Preferred is the use of the isosorbide derivatives inter alia in personal care compositions such as a liquid soap, shampoo, foam bath, shower bath and the like or a solid form such as a bar which can illustratively be a soap or syndet composition. The isosorbide derivatives could also be used in toothpaste and related compositions, like mouth wash. In addition to surfactants or surfactant combinations, the cosmetic products in question typically contain such constituents as emulsifiers, oil components, solubilizers, thickeners, superfatting agents, biogenic agents, film formers, fragrances, dyes, pearlescers, foam stabilizers, preservatives and pH regulators. Accordingly, the preparations according to the invention may contain additional components and auxiliaries as known from the prior art.

Any detergent or cleanser compositions according to the invention may contain, besides the isosorbide derivatives other surfactants, builders, salts, bleaching agents, bleach activators, optical brighteners, redeposition inhibitors, soil repellants, solubilizers, foam inhibitors, perfumes, buffers, non-aqueous solvents, dyes and enzymes as auxiliaries and additives.

The cleaners according to the invention may further contain, for example, solubilizers, such as ethanol, isopropyl alcohol, ethylene glycol, diethylene glycol or preferably butyl diglycol, foam regulators, for example soap, soluble builders, for example citric acid or sodium citrate, EDTA or NTA, and abrasives as auxiliaries. In many cases, an additional bactericidal effect is required so that the multipurpose cleaners may contain cationic surfactants or biocides, for example glucoprotamine. The cleaners according to the invention may be both alkaline pH>7.5) and acidic (pH<6.5). The isosorbide derivatives may be formulated with other surfactants, like anionic, nonionic, amphoteric and/or cationic surfactants.

Anionic surfactants according to the present invention include aliphatic sulfates, such as fatty alcohol sulfates, fatty alcohol ether sulfates, fatty acid polyglycol ester sulfates, dialkyl ether sulfates, monoglyceride sulfates and aliphatic sulfonates, such as alkane sulfonates, olefin sulfonates, ether sulfonates, n-alkyl ether sulfonates, ester sulfonates, and lignin sulfonates. Fatty acid cyanamides, sulfosuccinic acid esters, fatty acid isethionates, acylaminoalkane sulfonates (fatty acid taurides), fatty acid sarcosinates, ether carboxylic acids and alkyl (ether) phosphates may also be used for the purposes of the invention, but are not preferred. Preferred anionic surfactants in the sense of the present invention are selected from the group of fatty alcohol sulfates, fatty alcohol ether sulfates and/or fatty acid polyglycol ester sulfates, and mixtures thereof.

Typical examples of nonionic surfactants are alkoxylates of alkanols, end-capped alkoxylates of alkanols with no free OH groups, alkoxylated fatty acid lower alkyl esters, amine oxides, alkylphenol polyglycol ethers, fatty acid polyglycol esters, fatty acid amide polyglycol ethers, fatty amine polyglycol ethers, alkoxylated triglycerides, mixed ethers and mixed formals, fatty acid-N-alkyl glucamides, protein hydrolyzates (more particularly wheat-based vegetable products), polyol fatty acid esters, sugar esters, sorbitan esters and polysorbates. If the nonionic surfactants contain polyglycol ether chains, they may have a conventional homolog distribution although they preferably have a narrow homolog distribution. The other nonionic surfactants are preferably selected from the group consisting of alkoxylates of alkanols, more particularly fatty alcohol polyethylene glycol/polypropylene glycol ethers or fatty alcohol polypropylene glycol/polyethylene glycol ethers, end-capped alkoxylates of alkanols, more particularly end-capped fatty alcohol polyethylene glycol/polypropylene glycol ethers or end-capped fatty alcohol polypropylene glycol/polyethylene glycol ethers, and fatty acid lower alkyl esters and amine oxides.

Alkyl and alkenyl oligoglycosides are known, and preferred, nonionic surfactants which correspond to formula R-O-[G]ₚ in which R is an alkyl and/or alkenyl group containing 6 to 22 carbon atoms, G is a sugar unit containing 5 or 6 carbon atoms and p is a number of 1 to 10. They may be obtained by the relevant methods of preparative organic chemistry. The alkyl and/or alkenyl oligoglycosides may be derived from aldoses or ketoses containing 5 or 6 carbon atoms, preferably glucose. Accordingly, the preferred alkyl and/or alkenyl oligoglycosides are alkyl and/or alkenyl oligoglucosides. The index p in general formula (V) indicates the degree of oligomerization (DP), i.e. the distribution of mono- and oligoglycosides, and is a number of 1 to 10. Whereas p in a given compound must always be an integer and, above all, may assume a value of 1 to 6, the value p for a certain alkyl oligoglycoside is an analytically determined calculated quantity which is generally a broken number. Alkyl and/or alkenyl oligoglycosides having an average degree of oligomerization p of 1.1 to 3.0 are preferably used. Alkyl and/or alkenyl oligoglycosides having a degree of oligomerization of less than 1.7 and more particularly, between 1.2 and 1.4 are preferred from the applicational point of view. The alkyl or alkenyl group R may be derived from primary alcohols containing 4 to 11 and preferably 8 to 10 carbon atoms. Typical examples of cationic surfactants are quaternary ammonium compounds and quaternized fatty acid trialkanolamine esters. Typical examples of amphoteric or zwitterionic surfactants are alkyl betaines, alkyl amidobetaines, aminopropionates, aminoglycinates, imidazolinium betaines and sulfobetaines.

The phosphate esters according to the invention show, besides good detergency, also superior foaming properties, compared with other known surfactants, in particular to alkoxylated ether sulfates.

Foam properties of interest here are foam volume and tactile properties of the foam. The latter one is of specific advantage for the formulation of hand detergents, manual dish detergents, and personal care as well as cosmetic compositions. Preferred applications for these isosorbide derivatives may be in shampoos or shower gels and the like.

A particular advantage of the isosorbide phosphates is their capability to act as foaming surfactant in hard water, which is a water with a German hardness of below 5°, or even below 1° dH.

### Examples

### Preparation of an isosorbide mono-phosphate ester

### Example 1:

To a solution of 16.55 g isosorbide-mono-dodecylether (0.05 mol) in 200 ml of tetrahydrofuran (THF), 8.5 g P₄O₁₀ (0.06 mol) is added in portions at 80 °C and kept under reflux for additional 8 h. The product is then removed from the THF by distillation. In order to avoid the generation of unwanted phosphorous salts the crude product is solved in 200 ml of CHCl₃ and washed twice 50 ml of water. After removal of the CHCl₃ 24 g of acidic isosorbide mono-docecylether phosphates are obtained.

### Performance tests of the isosorbide phosphate esters

The isosorbide phosphate as prepared in example 1 was tested in comparison to C12/C14-fatty alcohol ethersulfate (2 EO) sold as Texapon^{®} LS 35 by the applicant, with regard to the foaming properties in a roto foam test with 0.5 g/l active substance (AS) in water at 40 °C. The pH of the aqueous solution was adjusted to 6.5 by the addition of sodium hydroxide. The isosorbide phosphate of example 1 show very similar advantageous foaming properties at 0° German hardness as the comparative product Texapon^{®} LS 35.

## Claims

1. The use of isosorbide phosphates according to general formula (I)
R-PO(OH)₂ (I)
and R is a compound according to the general formula (II) 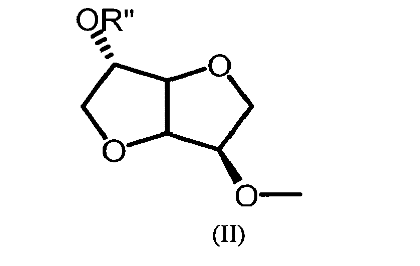 whereby in formula (II) R" represents a hydrogen atom or an alkyl- or alkenyl group with 1 to 22 C-atoms, which is linear or branched, saturated or unsaturated for the preparation of cleaners, detergents, personal care, cosmetic or pharmaceutical compositions, preferably in cleaners, personal care and cosmetic compositions.

2. Use according to claim 1, characterized that the isosorbide moiety according to formula (II) contains as group R" a linear alkyl group with 6 to 22 C-atoms.

3. The use according to claim 2 **characterized in that** the isosorbide phosphate ester is present in amounts from 0.1 to 25 wt%, preferably 1 to 15 wt%, and most preferred from 5 to 10 wt%, based on the total weight of the cleanser or detergent or personal care or cosmetic or pharmaceutical compositions.

4. The use according to one of the claims 1 to 3, characterized that the isosorbide phosphate ester is used as foaming surfactant for aqueous solutions with a German hardness below 5° ,preferably below 1° dH.
